# EUROPEAN PATENT APPLICATION

(11) **EP 3 741 407 A1**
(43) Date of publication of application: **25.11.2020**
(21) Application number: 19176370.5
(22) Date of filing: 24.05.2019
(51) Int. Cl.: A61M 11/04, A24F 47/00, A61M 15/06

(54) **AEROSOL DELIVERY DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery device comprises: a storage for storing an aerosol precursor; an aerosol generator comprising an aerosol generator portion configured to receive the aerosol precursor from the storage; an air flow passage configured to direct air past the aerosol generator portion to pick up the aerosol precursor from the aerosol generator portion to form an aerosol; and a barrier arrangement for inhibiting evaporation of aerosol precursor when the barrier arrangement is closed, the barrier arrangement being openable to permit generation of aerosol.

## Description

### Field of the Invention

The present invention relates to an aerosol delivery device and particularly, although not exclusively, to an aerosol delivery device in which a barrier arrangement inhibits evaporation of aerosol precursor.

### Background

A smoking-substitute device is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heating element. The consumable may also be referred to as a cartomizer. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu(RTM) e-cigarette. The myblu(RTM) e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices with passive aerosol generation, it is desirable to prevent evaporation of aerosol precursor when the device is not being used.

The present invention has been devised in light of the above considerations.

### Summary of the Invention

Generally this application relates to an aerosol delivery device in which a barrier arrangement inhibits evaporation of aerosol precursor when the barrier arrangement in closed, and the barrier arrangement is openable to permit generation of aerosol.

According to the present invention, there is provided aerosol delivery device comprising: a storage for storing an aerosol precursor; an aerosol generator comprising an aerosol generator portion configured to receive the aerosol precursor from the storage; an air flow passage configured to direct air past the aerosol generator portion to pick up the aerosol precursor from the aerosol generator portion to form an aerosol; and a barrier arrangement for inhibiting evaporation of aerosol precursor when the barrier arrangement is closed, the barrier arrangement being openable to permit generation of aerosol.

Such an aerosol delivery device is less prone to leakage during transit. Further, evaporation of the aerosol precursor can be prevented when the device is not in use.

Optionally, the barrier arrangement may be configured to open in response to a user inhaling on the aerosol delivery device.

Advantageously, the barrier arrangement may be selectively openable by a user.

Conveniently, the barrier arrangement may further comprise a mouthpiece and a body, wherein the barrier arrangement is configured to open in response to sliding of the mouthpiece relative to the body..

Optionally, the barrier arrangement may comprise a barrier element between the aerosol generator portion and a mouthpiece aperture of the aerosol delivery device to inhibit evaporation of aerosol precursor.

Advantageously, the barrier element may comprise a non-return valve.

Conveniently, the barrier element may comprise a movable shield.

Optionally, the barrier arrangement may be configured to inhibit flow of aerosol precursor between the storage and the aerosol generator portion to inhibit evaporation of aerosol precursor.

Advantageously, the aerosol delivery device may further comprise a transfer element, wherein the transfer element is movable with respect to the barrier arrangement to open the barrier arrangement so that the transfer element can transfer aerosol precursor from the storage to the aerosol generator portion.

Conveniently, the aerosol delivery device may further comprise a member, the member comprising the transfer element and the aerosol generator portion, wherein the member is movable from a first position to a second position to open the barrier arrangement.

Optionally, the aerosol delivery device may further comprise a supporting portion for maintaining a position of the member with respect to the mouthpiece during sliding of the mouthpiece.

Advantageously, the aerosol delivery device may further comprise: a pressure relief opening in the storage; and a blocking arrangement for inhibiting flow through the pressure relief opening, wherein the blocking arrangement is openable to permit air to flow through the pressure relief opening and into the storage as the storage empties of aerosol precursor.

Conveniently, the aerosol delivery device may be a consumable for a vaping device.

Optionally, the aerosol delivery device may further comprise an additional aerosol generator, the additional aerosol generator configured to produce an additional aerosol from an additional aerosol precursor.

Advantageously, the additional aerosol generator may be configured to heat the additional aerosol precursor to form the additional aerosol.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Fig. 1** shows a schematic drawing of a smoking substitute device;
**Fig. 2** shows a schematic drawing of a smoking substitute device;
**Fig. 3** shows a schematic drawing of a smoking substitute device;
**Fig. 4** shows a schematic drawing of a smoking substitute device;
**Fig. 5** shows a cross-sectional view of a consumable in a deactivated state;
**Fig. 6** shows a cross-sectional view of the consumable of Fig. 5 in an activated state;
**Fig. 7** shows a cross-sectional view of a flavour pod portion of a consumable;
**Fig. 8a** shows a top view of a flavour pod portion of a consumable;
**Fig. 8b** shows a cut away perspective view of a flavour pod portion of a consumable;
**Fig. 9** shows a cross-sectional view of a flavour pod portion of a consumable; and
**Fig. 10** shows a cross-sectional view of a flavour pod portion of a consumable.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

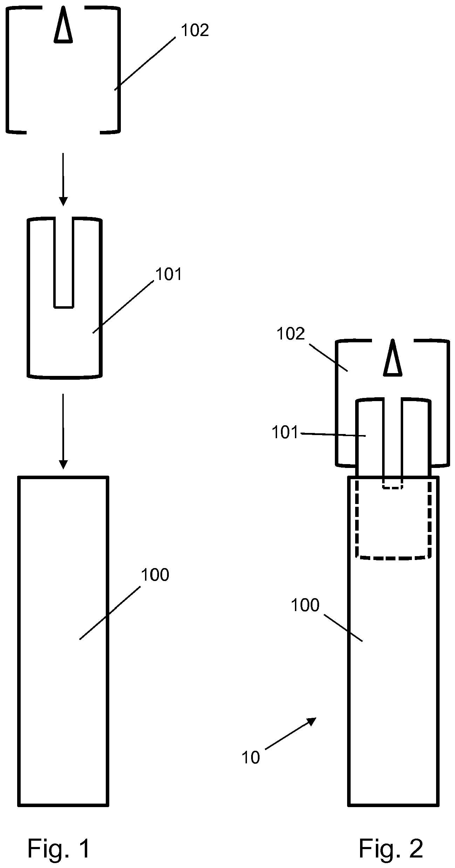

Referring to Figures 1 and 2, there is shown a smoking substitute device 10. In this example, the smoking substitute device comprises a cartomiser 101 and a flavour pod 102 connected to a base unit 100. In this example, the base unit 100 includes elements of the smoking substitute device such as a battery, an electronic controller, and a pressure transducer. The cartomiser 101 may engage with the base unit 100 via a push-fit engagement, a screw-thread engagement, or a bayonet fit, for example. A cartomiser may also be referred to as a "pod". The smoking substitute device can include an aerosol delivery device according to the present invention.

The flavour pod 102 is configured to engage with the cartomiser 101 and thus with the base unit 100. The flavour pod 102 may engage with the cartomiser 101 via a push-fit engagement, a screw-thread engagement, or a bayonet fit, for example. Fig. 2 illustrates the cartomiser 101 engaged with the base unit 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device according to the present invention.

As will be appreciated from the following description, the cartomiser 101 and the flavour pod 102 may alternatively be combined into a single component that implements the functionality of the cartomiser 101 10 and flavour pod 102. Such a single component may also be an aerosol delivery device according to the present invention. In other examples, the cartomiser may be absent, with only a flavour pod 102 present.

A "consumable" component may mean that the component is intended to be used once until exhausted, and then disposed of as waste or returned to a manufacturer for reprocessing.

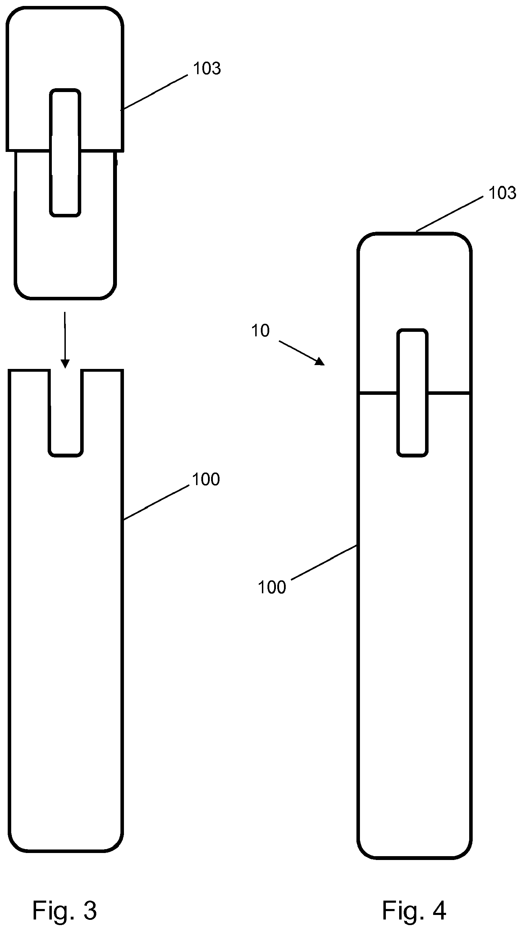

Referring to Figures 3 and 4, there is shown a smoking substitute device comprising a base unit 100 and a consumable 103. The consumable 103 combines the functionality of the cartomiser 101 and the flavour pod 102. In Figure 3, the consumable 103 and the base unit 100 are shown separated from one another. In Figure 4, the consumable 103 and the base unit 100 are engaged with each other to form the smoking substitute device 10.

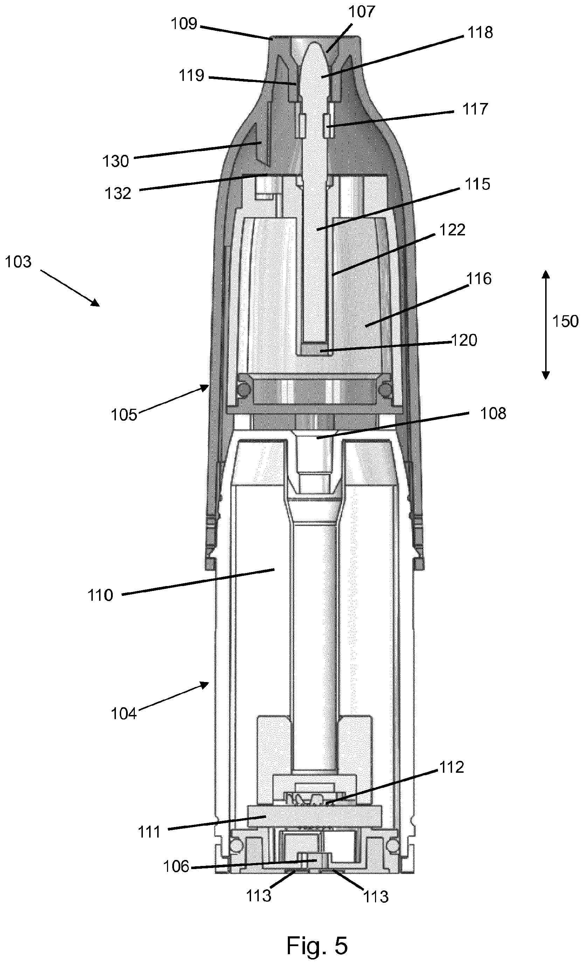

Referring to Figure 5, there is shown a consumable 103 engagable with a base unit via a push-fit engagement in a deactivated state. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a liquid transfer element 115, in the form of a member 115. The member 115 acts as a passive aerosol generator (e.g. an aerosol generator which does not use heat to form the aerosol, also referred to as a "first aerosol generator" in this example), and is formed of a porous material. The member 115 comprises a supporting portion 117, which is located inside a housing, and an aerosol generator portion 118, which is located in the airflow passage 108. In this example, the aerosol generator portion 118 is a porous nib.

When activated, as discussed in more detail below, a first storage 116 (in this example a tank) for storing a first aerosol precursor (i.e. a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage 116 into the member 115 via a wicking action.

Before activation, the barrier arrangement 120 is closed and inhibits evaporation of aerosol precursor. In this example, this is achieved by the barrier arrangement inhibiting flow of aerosol precursor from the first storage 116 to the member 115. In order to inhibit flow of aerosol precursor, the barrier arrangement 120 substantially isolates the first storage 116 from the member 115. In this example, the barrier arrangement comprises a plug 120 (preferably formed from silicon) located at one end of a tube 122 containing the member 115 close to the first storage 116. In other examples, the plug may be replaced by a deformable and/or breakable barrier component, e.g. any one of: a duck bill valve; a split valve or diaphragm; or a sheet of foil, which may be pierced by the member 115 when opening the barrier arrangement.

The first storage 116 further includes a pressure relief opening 132, which in the deactivated state is sealed by blocking arrangement. In this example, the blocking arrangement comprises a pierceable cover (preferably made from foil). Piercing member 130, which is formed as a part of the mouthpiece 109 and may take the form of a blade, pierces the pierceable cover and opens the pressure relief opening 132 when the consumable is moved to the activated state (as is discussed in more detail below). This means that opening of the barrier arrangement also effects opening of the blocking arrangement.

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increased air velocity and corresponding reduction in air pressure at the aerosol generator portion 118, the constriction is a Venturi aperture 119. The constriction is generally toroidal in shape, and may include one or more intersections where supports contact the aerosol generator portion 118.

The cartomiser portion 104 of the consumable 103 includes a second storage 110 (in this example a tank) for storing a second aerosol precursor (i.e. e-liquid, which may contain nicotine). Extending into the second storage 110 is a wick 111. The wick 111 is formed from a porous wicking material (e.g. a polymer) that draws second aerosol precursor from the second storage 110 into a central region of the wick 111 that is located outside the e-liquid storage tank 110.

A heater 112 is a configured to heat the central region of the wick 111. The heater 112 includes a resistive heating filament that is coiled around the central region of the wick 111. The wick 111, the heater 112 and the e-liquid storage tank 110 together act as an active aerosol generator (i.e. an aerosol generator which uses heat to form the aerosol, referred to as a "second aerosol generator" in this example).

As described above, the first and second aerosol generators are both at least partially located within the airflow passage 108, with the first aerosol generator downstream (with respect to air flow in use) of the second aerosol generator.

So that the consumable 103 may be supplied with electrical power for activation of the heater 112, the consumable 103 includes a pair of consumable electrical contacts 113. The consumable electrical contacts 113 are configured for electrical connection to a corresponding pair of electrical supply contacts in the base unit 100. The consumable electrical contacts 113 are electrically connected to the electrical supply contacts 114 when the consumable 103 is engaged with the base unit 100. The base unit 100 includes an electrical power source (not shown), for example a battery.

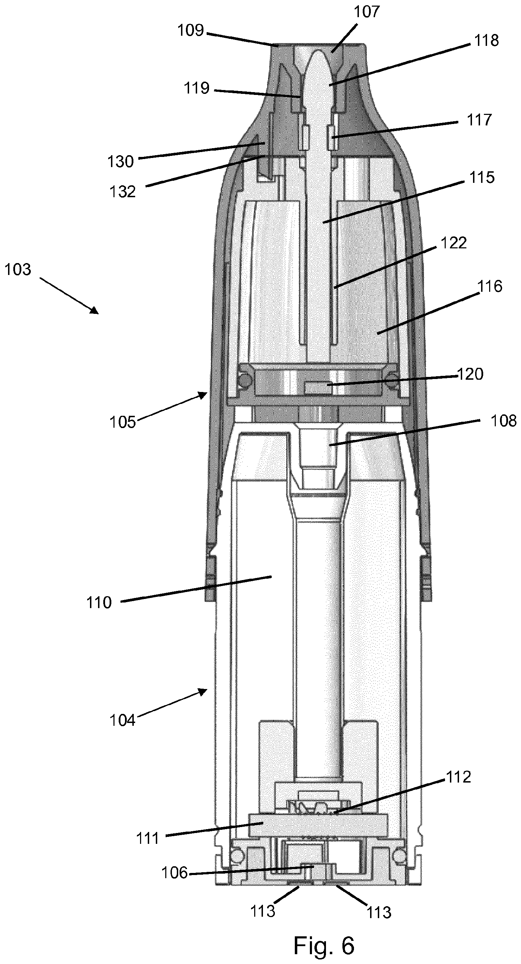

Figure 6 shows the consumable 103 of Figure 5 in an activated state, like features are indicated by like reference numerals. To transition from the deactivated state to the activated state, mouthpiece 109 is moved along a central axis 150 towards cartomizer portion 104 (e.g. one along which the consumable extends, and along which member 115 extends). Moving the mouthpiece 109 in this way effects relative movement between the liquid transfer element (i.e. the member 115) and the barrier arrangement. This causes the barrier arrangement to open. In other examples, a switch is provided for opening the barrier arrangement.

The mouthpiece 109, via supporting portion 117, is fixed to the member 115 and therefore member 115 moves with the mouthpiece 109. The mouthpiece 109, and member 115, is moved relative to the tank 116. This causes displacement of the plug 120 and opening of the barrier arrangement 116.

At the same time, movement of the mouthpiece 109 causes the piercing member 130 to contact and pierce pressure relief opening 132, thereby fluidly connecting the airflow passage 108 to an interior of the first storage 116. This permits air to flow into the first storage 116 as the first storage empties of aerosol precursor in use.

In the present example, once the barrier arrangement is open, the plug 120 is unconstrained within the first storage. However, in other cases, the plug 120 may be received by a guide for inhibiting return of the plug to the closed position after displacement of the plug. The guide may comprise a recess for receiving the plug 120.

In the present example, the barrier arrangement remains permanently open after opening, as the plug 120 does not return to the tube 122. However, in other examples, the barrier arrangement is selectively openable and closable by the user. This may be achieved by the plug (or another type of barrier arrangement) being fixed to an end portion of the member 115, such that the member 115 is selectively exposable to the first storage 116.

Once activated, and in use, a user draws (or "sucks", "pulls", or "puffs") on the mouthpiece 109 of the consumable 103, which causes a drop in air pressure at the outlet 107, thereby generating airflow through the inlet 106, along the airflow passage 108, out of the outlet 107 and into the user's mouth.

When the heater 112 is activated (by passing an electric current through the heating filament in response to the user drawing on the mouthpiece 109, the drawing of air may be detected by a pressure transducer) the e-liquid located in the wick 111 adjacent to the heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The base unit 100 supplies electrical current to the consumable electrical contacts 113. This causes an electric current flow through the heating filament of the heater 112 and the heating filament heats up. As described, the heating of the heating filament causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure and high air velocity region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol is sized to inhibit pulmonary penetration. The first aerosol is formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol is sized for transmission within at least one of a mammalian oral cavity and a mammalian nasal cavity. The first aerosol is formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

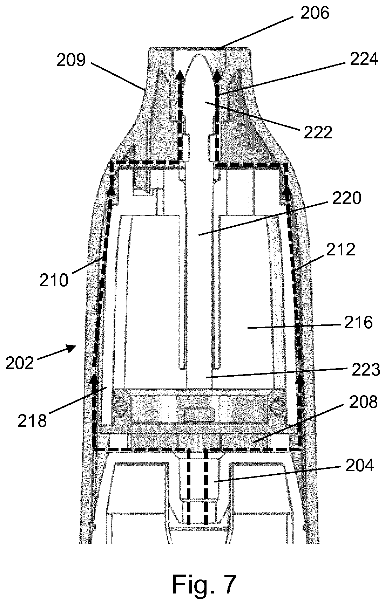

Referring to Fig. 7, there is shown a flavour pod portion 202 of a consumable in an activated state, the consumable providing an aerosol delivery device in accordance with the invention. The consumable further comprises a cartomiser portion (not shown in Fig. 7) having all of the features of the cartomiser portion 104 described above with respect to Figs. 5 and 6. However, in other examples, the consumable does not comprise the cartomiser portion, and provides only flavour to the user.

The flavour pod portion 202 comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly connecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage 214, which stores a first aerosol precursor. The storage 214 comprises a reservoir 216 located within a chamber 218. The reservoir 216 is formed of a first porous material.

The flavour pod portion 202 comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the storage 214. The member 220 is in contact with the reservoir 216. More specifically, the supporting portion 223 extends into and through the storage 204 and is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

In use, when a user draws on the mouthpiece 209, air flow is generated through the air flow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

As the first aerosol precursor is picked up by the air, the member 220 transfers further first aerosol precursor from the storage 214 to the aerosol generator portion 222. More specifically, the member 220 wicks the first aerosol precursor from the storage 214 to the aerosol generator portion 224.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 224.

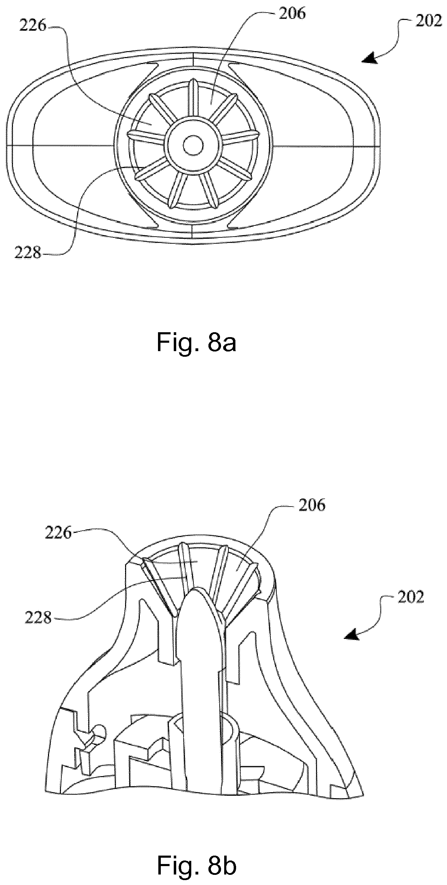

Figs. 8a and 8b show further views of the flavour pod portion 202 which highlight features of the mouthpiece 209. Many of the reference numerals of Fig. 7 are omitted from Fig. 8a and 8b for clarity.

The mouthpiece aperture 206 comprises an inner surface 226, which is uneven. In the present example, the inner surface 226 has the form of a substantially frustoconical surface, but includes grooves or channels 228 to make the inner surface 226 somewhat uneven. In other examples, the inner surface 226 may have another form (for example, the form a substantially cylindrical surface), and may include any type of protrusion or groove to make the inner surface uneven.

The inner surface 226 is angled with respect to an axial direction (i.e. relative to a central axis extending from a base of the consumable to the mouthpiece) such that the width of the mouthpiece aperture 209 increases in the downstream direction. The inner surface 226 is immediately downstream of the narrowing section 224 of the airflow passage 108.

The grooves 228 are generally v-shaped in cross-sectional profile, and extend in the axial direction for the full length of the inner surface 226. Each groove 228 is formed from a pair of surfaces angled at between 30 and 90 degrees relative to each other. More specifically, each groove 228 is formed from a pair of surfaces angled at 60 degrees relative to each other.

The grooves 228 have a depth (measured normal to the inner surface 226) of at least 0.2 mm. More specifically, the grooves 228 have a depth of at least 0.3 mm. More specifically, the grooves 228 have a depth of at least 0.4 mm.

The grooves 228 have a depth of less than 0.8 mm. More specifically, the grooves have a depth of less than 0.7 mm. More specifically, the grooves have a depth of less than 0.6 mm.

More specifically, the grooves have a depth of substantially 0.5 mm.

The grooves 228 are substantially equi-spaced in a circumferential manner around the inner surface 226. The inner surface 226 comprises at least 6 grooves. More specifically, the inner surface comprises at least 7 grooves. More specifically, the inner surface 226 comprises at least 8 grooves.

The inner surface 226 comprises at most 12 grooves 228. More specifically, the inner surface 226 comprises at most 11 grooves 228. More specifically, the inner surface 226 comprises at most 10 grooves 228.

More specifically, the inner surface 226 comprises 9 grooves 228.

The grooves 228 are spaced apart from each other by substantially 1 mm at the downstream end of the inner surface 226. In other examples, the spacing at the downstream end of grooves or protrusions may be selected such that it is equal to or less than the mass median diameter (as described above) of particles in the first aerosol.

The inner surface 226 comprises a smooth polished surface between the grooves 228. Polishing the surface in this way provides improved aerodynamic properties. However, in other examples, the inner surface 226 may be textured. In such examples, the texture of the surface may provide the uneven surface, and no grooves are required,

In use, the uneven nature of the inner surface 226 makes it easier for droplets to form on the inner surface 226, preventing large droplets from entering the user's mouth. The grooves 228 help to channel the large droplets back into the consumable.

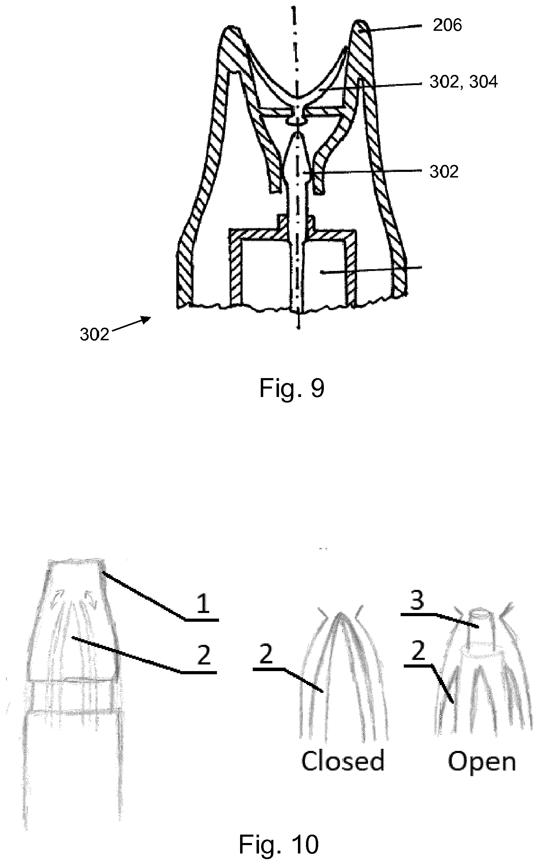

Referring to Fig. 9 there is shown a sectional drawing of a second flavour pod portion 302. The second flavour pod portion 302 comprises all of the features of the flavour pod portion 202 aside from the differences described here. Many of the reference numerals relating to features which are common between the second flavour pod portion 302 and the flavour pod portion 202 are omitted from Fig. 9 for clarity. However, like reference numerals are used in Fig. 9 where features referred to previously are referred to again.

The second flavour pod portion 302 comprises a second barrier arrangement 304 for inhibiting evaporation of aerosol precursor when the second barrier arrangement 304 is closed. The second barrier arrangement 302 does not inhibit flow between the first storage and the member 115, which means that the member 115 contains aerosol precursor even when the second barrier arrangement 304 is closed. The second barrier arrangement 304 comprises a valve 304 which is located in the outlet 206 of the second flavour pod portion 302. The valve 304 is a diaphragm valve. The valve 304 substantially seals the outlet 206.

In use, the user inhales on the mouthpiece, which causes the second barrier arrangement 302 to open, permitting aerosol to flow out of the outlet 206. The second barrier arrangement 302 closes when the user stops inhaling.

Referring to Fig. 10 there is shown a sectional drawing of a third flavour pod portion 402. The third flavour pod portion 402 comprises all of the features of the flavour pod portion 202 aside from the differences described here. Many of the reference numerals relating to features which are common between the second flavour pod portion 402 and the flavour pod portion 202 are omitted from Fig. 10 for clarity. However, like reference numerals are used in Fig. 10 where features referred to previously are referred to again.

The third flavour pod portion 402 comprises a third barrier arrangement 404 for inhibiting evaporation of aerosol precursor when the third barrier arrangement 404 is closed. As with the second barrier arrangement 302, the third barrier arrangement 402 does not inhibit flow between the first storage and the member 115, which means that the member 115 contains aerosol precursor even when the third barrier arrangement 404 is closed. The third barrier arrangement 404 is a shield 404. The shield 404 is formed of a plastic material. The shield 404 encloses the member 115 when the shield 404 is closed.

The shield 404 comprises four curved plates 406. When the shield 404 opens, the curved plates 406 separate and slide to expose the member 115 to permit generation of aerosol.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device comprising:
a storage for storing an aerosol precursor;
an aerosol generator comprising an aerosol generator portion configured to receive the aerosol precursor from the storage;
an air flow passage configured to direct air past the aerosol generator portion to pick up the aerosol precursor from the aerosol generator portion to form an aerosol; and
a barrier arrangement for inhibiting evaporation of aerosol precursor when the barrier arrangement is closed, the barrier arrangement being openable to permit generation of aerosol.

2. An aerosol delivery device according to claim 1, wherein the barrier arrangement is configured to open in response to a user inhaling on the aerosol delivery device.

3. An aerosol delivery device according to claim 1 or claim 2, wherein the barrier arrangement is selectively openable by a user.

4. An aerosol delivery device according to claim 3, and further comprising a mouthpiece and a body, wherein the barrier arrangement is configured to open in response to sliding of the mouthpiece relative to the body.

5. An aerosol delivery device according to any one of the preceding claim, wherein the barrier arrangement comprises a barrier element between the aerosol generator portion and a mouthpiece aperture of the aerosol delivery device to inhibit evaporation of aerosol precursor.

6. An aerosol delivery device according to claim 5, wherein the barrier element comprises a non-return valve.

7. An aerosol delivery device according to claim 5, wherein the barrier element comprises a movable shield.

8. An aerosol delivery device according to any one of claims 1 to 4, wherein the barrier arrangement is configured to inhibit flow of aerosol precursor between the storage and the aerosol generator portion to inhibit evaporation of aerosol precursor.

9. An aerosol delivery device according to claim 8 and further comprising a transfer element, wherein the transfer element is movable with respect to the barrier arrangement to open the barrier arrangement so that the transfer element can transfer aerosol precursor from the storage to the aerosol generator portion.

10. An aerosol delivery device according to claim 8 and further comprising a member, the member comprising the transfer element and the aerosol generator portion, wherein the member is movable from a first position to a second position to open the barrier arrangement.

11. An aerosol delivery device according to claim 10 when dependent on claim 4, and further comprising a supporting portion for maintaining a position of the member with respect to the mouthpiece during sliding of the mouthpiece.

12. An aerosol delivery device according to any one of the preceding claims, and further comprising:
a pressure relief opening in the storage; and
a blocking arrangement for inhibiting flow through the pressure relief opening,
wherein the blocking arrangement is openable to permit air to flow through the pressure relief opening and into the storage as the storage empties of aerosol precursor.

13. An aerosol delivery device according to any one of the preceding claims, wherein the aerosol delivery device is a consumable for a vaping device.

14. An aerosol delivery device according to any one of the preceding claims and further comprising an additional aerosol generator, the additional aerosol generator configured to produce an additional aerosol from an additional aerosol precursor.

15. An aerosol delivery device according to claim 14, wherein the additional aerosol generator is configured to heat the additional aerosol precursor to form the additional aerosol.
